# EUROPEAN PATENT APPLICATION

(11) **EP 4 721 666 A1**
(43) Date of publication of application: **08.04.2026**
(21) Application number: 24815530.1
(22) Date of filing: 29.05.2024
(51) Int. Cl.: A61B 5/308, A61B 5/352, A61B 5/339, A61B 5/361, A61B 5/00

(54) **ELECTROCARDIOGRAPH**

(30) Priority: 02.06.2023 JP 2023091405
(71) Applicant: Nipro Corporation, Settsu-shi, Osaka 566-8510 (JP); Harada Electronics Industry Co., Ltd., Sapporo-shi, Hokkaido 063-0052 (JP)
(72) Inventor: SANO, Yoshihiko, Settsu-shi, Osaka 566-8510 (JP); HARADA, Masahide, Sapporo-shi, Hokkaido 063-0052 (JP)
(74) Representative: Hafner & Kohl PartmbB
(86) International application number: PCT/JP2024/019731
(87) International publication number: WO 2024/248049

(57) **Abstract**

This invention makes high-precision electrocardiographic data analysis possible by recording electrocardiographic data with sufficiently expanded heartbeat waveforms in addition to recording electrocardiograms in accordance with a standard. An electrocardiograph of this invention includes:
a normal filter that is configured to extract electrocardiographic data of a normal frequency range based on an electrocardiogram standard out of electrocardiographic data detected by a bioelectrode and an indifferent electrode;
a normal channel data processing means that is configured to continuously record and output, for a long period of time, the electrocardiographic data which are of the normal frequency range and are extracted by the normal filter;
an additional filter that is configured to eliminate at least baseline drift artifacts caused by body motion by extracting, out of the electrocardiographic data detected by the bioelectrode and the indifferent electrode, electrocardiographic data of a given frequency range whose lower limit frequency is higher than that of the normal frequency range; and
an additional channel data processing means that is configured to continuously record and output, for a long period of time, the electrocardiographic data which are of the given frequency range and are extracted by the additional filter.

## Description

### Technical Field

This invention relates to an electrocardiograph that is configured to continuously record, based on an electrocardiogram standard, electrocardiographic data detected by a bioelectrode and an indifferent electrode for a long period of time, and is capable of expanding the heartbeat waveforms of such electrocardiographic data recorded.

### Background Art

As a conventional electrocardiograph that is configured to continuously record, based on an electrocardiogram standard, electrocardiographic data detected by a bioelectrode and an indifferent electrode for a long period of time, there is known, for example, a Holter electrocardiograph disclosed in Patent literature 1. This Holter electrocardiograph makes it possible to input and record events that occur while recording a Holter electrocardiogram, and allows editing of events and electrocardiograms at the time of playing back the Holter electrocardiogram recorded, thereby making comprehension of test results easy.

Here, as an electrocardiogram standard for a Holter electrocardiograph, there is known JIS-T-60601-2-47:2018. The frequency characteristic according to this electrocardiogram standard satisfies a condition where an amplitude with respect to a sine-wave signal of 2 mVp-v at 0.67 to 40 Hz is in a range of 70 to 140% (-3 dB to +3 dB), provided that an amplitude at 5 Hz is 100%. Particularly, the frequency characteristic for a Holter electrocardiograph is normally 0.05 to 100 Hz.

### Citation List

### Patent Literature

Patent Literature 1: JP-A-H04-352940

### Summary of Invention

### Technical Problem

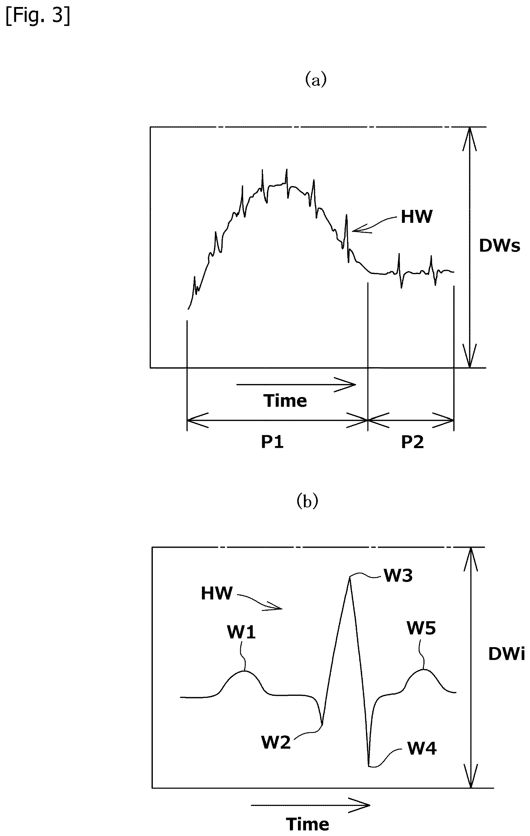

In the meantime, if taking an actual condition of use into consideration, as shown by an artifact-present period P1 in FIG.3(a), electrocardiographic data contain artifacts such baseline drifts that are caused by body motions including breathing or the like; as an electrocardiogram, such artifact-containing electrocardiographic data end up being recorded. For this reason, artifacts occupy a major part of a data width (largest data width usable for electrocardiographic data) DWs (e.g., 4096 digits) of the dynamic range of electrocardiographic data required by an electrocardiogram standard, whereby a data width DWi of each heartbeat waveform HW that is usable for electrocardiogram analysis and is shown in FIG.3(b) is thus inevitably reduced to a fraction (e.g., about 400 digits) of the data width DWs of the dynamic range of the electrocardiographic data, as indicated by an artifact-absent period P2 in FIG.3(a).

As shown in FIG.3(b), each heartbeat waveform HW is usually composed of a series of waveforms which are a waveform W1 called P wave, a waveform W2 called Q wave, a waveform W3 called R wave, a waveform W4 called S wave, and a waveform W5 called T wave. However, as described above, since the data width DWi of the heartbeat waveform HW is merely a fraction of the data width DWs of the dynamic range of the electrocardiographic data, it is often difficult to distinguish a small signal such as the P wave, in particular, as the first waveform W1 out of the heartbeat waveform HW from noises in the artifacts.

Thus, it is an object of this invention to provide an electrocardiograph that makes high-precision electrocardiographic data analysis possible by recording electrocardiographic data with sufficiently expanded heartbeat waveforms in addition to recording electrocardiograms in accordance with a standard.

### Solution of Problem

The electrocardiograph of this invention that is capable of advantageously solving the above problem, includes:
a normal filter that is configured to extract electrocardiographic data of a normal frequency range based on an electrocardiogram standard out of electrocardiographic data detected by a bioelectrode and an indifferent electrode;
a normal channel data processing means that is configured to continuously record and output, for a long period of time, the electrocardiographic data which are of the normal frequency range and are extracted by the normal filter;
an additional filter that is configured to eliminate at least baseline drift artifacts caused by body motion by extracting, out of the electrocardiographic data detected by the bioelectrode and the indifferent electrode, electrocardiographic data of a given frequency range whose lower limit frequency is higher than that of the normal frequency range; and
an additional channel data processing means that is configured to continuously record and output, for a long period of time, the electrocardiographic data which are of the given frequency range and are extracted by the additional filter.

### Advantageous Effects of Invention

In the case of the electrocardiograph of this invention, the normal filter is configured to extract the electrocardiographic data of the normal frequency range based on the electrocardiogram standard out of the electrocardiographic data detected by the bioelectrode and the indifferent electrode; and the normal channel data processing means is configured to continuously record and output, for a long period of time, the electrocardiographic data which are of the normal frequency range and are extracted by the normal filter. Further, the additional filter is configured to eliminate at least the baseline drift artifacts caused by body motion by extracting, out of the electrocardiographic data detected by the bioelectrode and the indifferent electrode, the electrocardiographic data of the given frequency range whose lower limit frequency is higher than that of the normal frequency range; and the additional channel data processing means is configured to continuously record and output, for a long period of time, the electrocardiographic data which are of the given frequency range and are extracted by the additional filter.

Thus, according to the electrocardiograph of this invention, there can be obtained, from the output signals of the normal channel data processing means, a normal electrocardiogram such as a 24-hour Holter electrocardiogram based on an electrocardiogram standard. In addition, from the output signals of the additional channel data processing means, even when the dynamic range is the same as that of the normal channel data processing means, e.g., a data width of 4,096 digits, there can still be obtained a heartbeat waveform having a data width of, for example, approximately 3,000 digits, such heartbeat waveform being that expanded by using the portion of the data width occupied by at least the baseline drift artifacts caused by body motion, thereby allowing each heartbeat waveform to be displayed in a large size and with a high precision.

Here, the electrocardiograph of this invention may also be such that
by extracting, out of the electrocardiographic data detected by the bioelectrode and the indifferent electrode, the electrocardiographic data of the given frequency range whose lower limit frequency is higher than that of the normal frequency range, the additional filter is further configured to at least extract the R wave of each heartbeat waveform in addition to eliminating the baseline drift artifacts caused by body motion, and
the additional channel data processing means is further configured to immediately record and output an atrial fibrillation alert signal upon detecting a variation in intervals between the R waves extracted in addition to continuously recording and outputting, for a long period of time, the electrocardiographic data which are of the given frequency range and are extracted by the additional filter.

In this way, variations in R-wave intervals can be reliably detected from high-precision heartbeat waveforms with expanded data widths, whereby upon reliably detecting atrial fibrillation occurring in the heart of a subject, an alert signal can be immediately output via, for example, an alarm sound and/or screen display on a smartphone.

### Brief Description of Drawings

[FIG.1] is a block diagram showing a first embodiment of the electrocardiograph of this invention.
[FIG.2] is a block diagram showing a second embodiment of the electrocardiograph of this invention.
[FIG.3] (a) is a relation diagram showing a relation between the passage of time and the level of electrocardiographic data of a normal channel of a Holter electrocardiograph in an actual condition of use; and (b) is a relation diagram showing a relation between the passage of time and the level of electrocardiographic data recorded and output by the additional channel data processing means in the electrocardiograph of the first embodiment.

### Description of Embodiments

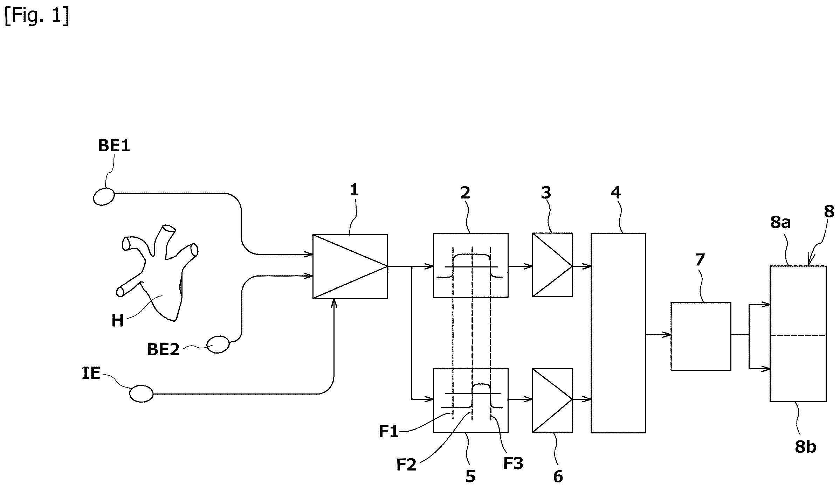

Embodiments of this invention are described in detail hereunder with reference to drawings. FIG.1 is a block diagram showing a first embodiment of an electrocardiograph of this invention, in which a symbol "H" in FIG.1 represents a heart of a subject from which electrocardiograms are to be obtained, symbols "BE1" and "BE2" represent bioelectrodes that are to be attached to the skin close to the subject's heart H, and a symbol "IE" represents an indifferent electrode that is to be attached to the skin close to the subject's heart H at a position substantially equally distant from the bioelectrodes BE1 and BE2.

As shown in FIG.1, the electrocardiograph of the first embodiment includes a bioamplifier 1 that is configured to amplify electrocardiographic data detected by the bioelectrodes BE1 and BE2 and the indifferent electrode IE, and then output such data as analog signals; a normal filter 2 that is configured to remove, from the electrocardiographic data output by the bioamplifier 1, signals of frequencies not higher than F1 and frequencies not lower than F3 based on a normal electrocardiogram standard such as the 24-hour Holter electrocardiogram standard, and then output the remaining electrocardiographic data; an amplifier 3 that is configured to amplify the electrocardiographic data output by the normal filter 2 at a low gain corresponding to a data width DWs of the dynamic range of the electrocardiographic data to be recorded, and then output the amplified data; and an A/D convertor 4 that is configured to convert the analog signals of the electrocardiographic data output by the amplifier 3 into digital signals with, for example, a sampling period of 4 ms, and then output such digital signals. Here, in the first embodiment, for example, based on the 24-hour Holter electrocardiogram standard, a frequency F1 is defined as 0.05 Hz, and a frequency F3 is defined as 60 Hz.

Further, the electrocardiograph of the first embodiment includes a first additional filter 5 that is configured to remove, from the electrocardiographic data output by the bioamplifier 1, signals of frequencies not higher than F2 and frequencies not lower than F3, and then output the remaining electrocardiographic data; and an amplifier 6 that is configured to amplify the electrocardiographic data output by the first additional filter 5 at such a high gain that a dada width DWi of a heartbeat waveform HW to be recorded is able to be expanded close to the data width DWs of the dynamic range of the electrocardiographic data, and then output the amplified data. The analog signals of such electrocardiographic data output by the amplifier 6 are also converted into digital signals by the A/D convertor 4 with, for example, a sampling period of 4 ms before being output therefrom. Here, in the first embodiment, a frequency F2 is defined as 10 Hz so that baseline drift artifacts caused by body motion are able to be removed from the electrocardiographic data detected by the bioelectrodes BE1 and BE2 and the indifferent electrode IE.

Moreover, the electrocardiograph of the first embodiment includes a CPU (central processing unit) 7. Based on a previously given program, the CPU 7 is configured to control the operation of the A/D convertor 4 such that the analog signals of the electrocardiographic data output by the amplifier 3 and the amplifier 6 are converted into digital signals with, for example, the sampling period of 4 ms, and then output the electrocardiographic data of such digital signals output by the A/D convertor 4 to a storage circuit 8. The storage circuit 8 is configured to store (record) the electrocardiographic data from the amplifier 3 out of the above electrocardiographic data in a storage element such as a memory of a normal channel storage circuit 8a in a readable manner, and store (record) the electrocardiographic data from the amplifier 6 out of the above electrocardiographic data in a storage element such as a memory of an additional channel storage circuit 8b in a readable manner. Here, it is preferable to read out the electrocardiographic data from the storage elements of the normal channel storage circuit 8a and the additional channel storage circuit 8b with a magnetic circuit that is configured by stacking together a pair of coils in a mutually separable manner, because the read-out can be carried out in a compact and non-contact fashion.

That is, according to the electrocardiograph of the first embodiment, while the amplifier 3, the A/D convertor 4, the CPU 7, and the normal channel storage circuit 8a correspond to a normal channel data processing means, the amplifier 6, the A/D convertor 4, the CPU 7, and the additional channel storage circuit 8b correspond to an additional channel data processing means. As shown in FIG.3(a), there can be obtained a normal electrocardiogram based on a normal electrocardiogram standard such as the 24-hour Holter electrocardiogram standard; and as shown in FIG.3(b), even when the dynamic range is the same as that of the normal channel data processing means, e.g., a data width of 4,096 digits, there can still be obtained a heartbeat waveform having a data width of, for example, approximately 3,000 digits, such heartbeat waveform being that expanded by using the portion of the data width occupied by at least the baseline drift artifacts caused by body motion, thereby allowing each heartbeat waveform to be displayed in a large size and with a high precision. Here, although this expanded heartbeat waveform may be distorted to a certain extent as compared to before the expansion, a P wave can still be displayed in an expanded manner with a positional relationship between the P wave as a wave form W1 and an R wave as a wave form W3 remaining substantially unchanged.

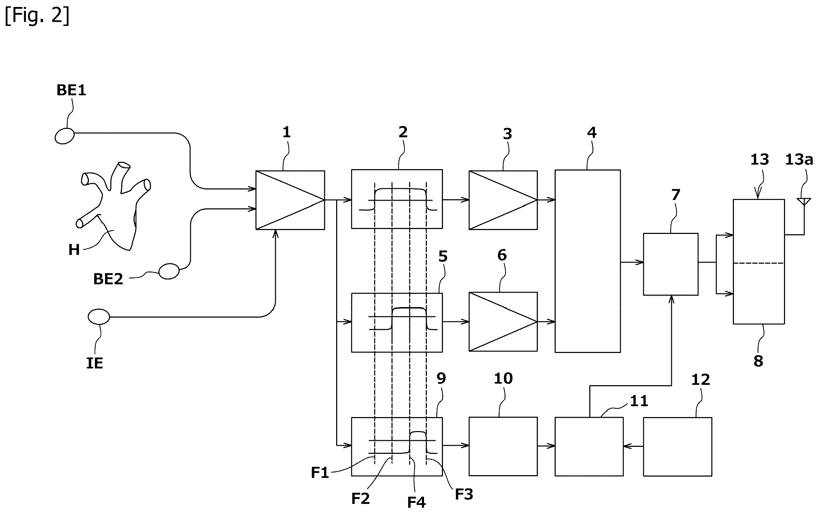

FIG.2 is a block diagram showing a second embodiment of the electrocardiograph of this invention; in FIG.2, parts similar to those shown in FIG.1 are indicated by corresponding identical symbols.

In addition to the configuration of the electrocardiograph of the first embodiment shown in FIG.1, the electrocardiograph of the second embodiment shown in FIG.2 further includes a second additional filter 9, an R-wave detector 10, a counter 11, an R-R measurement signal generator 12, and a signal input and output part 13. The second additional filter 9 is configured to output electrocardiographic data with the R wave of each heartbeat waveform HW being enhanced, such electrocardiographic data being what remained after removing, from the electrocardiographic data output from the bioamplifier 1, signals of frequencies not higher than F4 (e.g., 20 Hz) and frequencies not lower than F3 (e.g., 60 Hz). The R-wave detector 10 is configured to detect the rise of the R wave of each heartbeat signal of the electrocardiographic data output by the second additional filter 9, and output a pulse signal(s) synchronized with such rise. The counter 11 is configured to measure an R-R interval of the above pulse signals corresponding to the R waves of neighboring heartbeat waveforms HW when supplied with an R-R measurement signal that is provided from the R-R measurement signal generator 12 and has a frequency higher than that of the above sampling period, and then send such R-R interval measured to the CPU7.

The CPU 7 is configured to continuously calculate, for every given period, a root mean square (RMS) of a difference between the latest R-R interval measured and a previously measured R-R interval, and determine whether the R-R intervals vary so significantly that the RMS is as large as or larger than a given threshold value, where if the R-R intervals are determined as varying significantly, the CPU 7 will output an atrial fibrillation alert signal to the signal input and output part 13 such that the signal input and output part 13 will immediately output an alert signal via, for example, an alarm sound from a speaker that is not shown and/or smartphone screen displaying that is effected by radio waves from an antenna 13a. Further, the signal input and output part 13 may perform the input and output of signals between the CPU 7 and, for example, an external personal computer via, for example, a connector that is not shown, whereby the operation of the CPU 7 can be controlled by a control program or the like input from the external personal computer, and the normal electrocardiographic data as well as the electrocardiographic data from which artifacts have been removed, which are stored in the storage circuit 8, can be output to and screen-displayed on the external personal computer.

Thus, according to the electrocardiograph of the second embodiment, in addition to the function effect of the above electrocardiograph of the first embodiment, the electrocardiograph of the second embodiment further has a function effect where it is capable of reliably detecting variations in R-wave intervals from high-precision heartbeat waveforms with expanded data widths, whereby as an atrial fibrillation detector (AF detector), atrial fibrillation occurring in the heart of a subject can thus be reliably detected, and an alert signal can be immediately output via, for example, an alarm sound and/or screen display on a mobile terminal such as a smartphone. Here, the detection of the variations in R-R intervals may be performed by a different method instead of the method using root mean square (RMS).

The invention has thus far been described based on the embodiments illustrated in the drawings. However, the present invention shall not be limited to the above embodiments, but may be appropriately modified within the scope described in the claims. For example, the electrocardiogram standard employed by the normal filter and the normal channel data processing means shall not be limited to the 24-hour Holter electrocardiogram standard, but may also be one dedicated to continuously recording electrocardiograms for an even longer period of time. Further, in the second embodiment, it may be that high-frequency signals in the CPU 7 are used instead of the R-R measurement signal generator 12. Furthermore, the additional filter(s) may be configured to perform only the extraction of R waves unlike the above first and second embodiments with the specific frequency ranges through which the electrocardiographic data pass.

### Industrial Applicability

In this way, according to the electrocardiograph of this invention, not only a normal electrocardiogram such as a 24-hour Holter electrocardiogram based on an electrocardiogram standard can be obtained from the output signal of the normal channel data processing means, but there can also be obtained, from the output signal of the additional channel data processing means, a heartbeat waveform having a data width that has been expanded by using the portion of the data width occupied by at least the baseline drift artifacts caused by body motion, even when the dynamic range is of the same data width as that of the normal channel data processing means, thereby allowing each heartbeat waveform to be displayed in a large size and with a high precision.

### Description of symbols

1 Bioamplifier
2 Normal filter
3 Amplifier
4 A/D convertor
5 First additional filter
6 Amplifier
7 CPU
8 Storage circuit
8a Normal channel storage circuit
8b Additional channel storage circuit
9 Second additional filter
10 R-wave detector
11 Counter
12 R-R measurement signal generator
13 Signal input and output part
13a Antenna
BE1, BE2 Bioelectrode
DWi Data width of heartbeat waveform
DWs Data width of dynamic range of electrocardiographic data
IE Indifferent electrode
H Heart
HW Heartbeat waveform
P1 Artifact-present period
P2 Artifact-absent period
W1 P wave
W2 Q wave
W3 R wave
W4 S wave
W5 T wave

## Claims

1. An electrocardiograph comprising:
a normal filter that is configured to extract electrocardiographic data of a normal frequency range based on an electrocardiogram standard out of electrocardiographic data detected by a bioelectrode and an indifferent electrode;
a normal channel data processing means that is configured to continuously record and output, for a long period of time, the electrocardiographic data which are of the normal frequency range and are extracted by the normal filter;
an additional filter that is configured to eliminate at least baseline drift artifacts caused by body motion by extracting, out of the electrocardiographic data detected by the bioelectrode and the indifferent electrode, electrocardiographic data of a given frequency range whose lower limit frequency is higher than that of the normal frequency range; and
an additional channel data processing means that is configured to continuously record and output, for a long period of time, the electrocardiographic data which are of the given frequency range and are extracted by the additional filter.

2. The electrocardiograph according to claim 1, wherein
by extracting, out of the electrocardiographic data detected by the bioelectrode and the indifferent electrode, the electrocardiographic data of the given frequency range whose lower limit frequency is higher than that of the normal frequency range, the additional filter is further configured to at least extract the R wave of each heartbeat waveform in addition to eliminating the baseline drift artifacts caused by body motion, and
the additional channel data processing means is further configured to immediately record and output an atrial fibrillation alert signal upon detecting a variation in intervals between the R waves extracted in addition to continuously recording and outputting, for a long period of time, the electrocardiographic data which are of the given frequency range and are extracted by the additional filter.

3. The electrocardiograph according to claim 2, wherein the additional channel data processing means is further configured to output the atrial fibrillation alert signal via at least one of an alarm sound and screen display on a mobile terminal.
